# EUROPEAN PATENT APPLICATION

(11) **EP 4 438 071 A1**
(43) Date of publication of application: **02.10.2024**
(21) Application number: 23165170.4
(22) Date of filing: 29.03.2023
(51) Int. Cl.: A61M 1/16, A61M 1/28, A61M 1/34, A61M 27/00

(54) **MEDICAL SYSTEM FOR SUPPORTING NATIVE KIDNEY FUNCTION**

(71) Applicant: Fraunhofer-Gesellschaft zur Förderung der angewandten Forschung e.V., 80686 München (DE)
(72) Inventor: Goldau, Dr. Rainer, 04103 Leipzig (DE); Salti, Haitham, 04103 Leipzig (DE)
(74) Representative: Pfenning, Meinig & Partner mbB

(57) **Abstract**

The present disclosure describes a medical system (600) for supporting native kidney function of a patient's kidney, which comprises a fluid source (602) for providing fluid to be injected into the patient's kidney, a fluid outlet (616) in fluid connection with the fluid source for transferring the fluid (608) to a renal cortex of the patient's kidney, and a control unit (620) that receives physiological condition information (622) indicative of a physiological condition of the patient and that is configured to control a flow through the fluid outlet (616) using the physiological condition information (622).

## Description

The present disclosure relates to a medical system for supporting native kidney function of a patient's kidney.

Several conditions, acute and chronic, can lead to kidney malfunction or failure. In case of a chronic disease, organ transplantation to replace the kidney is possible. However, donor organs are scarce such that at the end, usually all patients, at least for some time are required to undergo a dialysis treatment. In most cases, patients have to undergo hemodialysis. Hemodialysis directly accesses the bloodstream and removes uremic toxins. However, hemodialysis shows a number of shortcomings that have to be addressed yet. The commonly used dialysis techniques remove uremic toxins through diffusion. For this reason, hemodialysis requires a continuous supply of fresh dialysate, which requires patients to regularly visit dialysis facilities for multiple hours per week. Those required regular visits of dialysis facilities put an enormous pressure on the patient's work and social life. Moreover, hemodialysis requires large amounts of fluid being sourced from a reverse osmosis (RO) unit supply as a basis for the dialysate required for the hemodialysis. In addition, current hemodialysis techniques do not only remove uremic toxins, but also valuable ingredients like electrolytes and bicarbonate from the patient's blood. Admixing these ingredients to the RO-water administered requires additional effort and in those cases in which the patient's intrinsic values are not met it poses additional burden to the patient.

Different approaches have been introduced to address those short-comings of hemodialysis. In the case of the discarded water, EP 3487555B1, for example, proposes to use the cleaning effect of freezing water. Ice crystals exclude any contaminations when they form and only need to be washed externally. The own melting water can be used for this purpose within a wash column. This solution at least can solve the water problem of dialysis by generating fresh water and a drain aliquot concentrated to an extent that permits refilling this volume from the patient's own body water without dehydration. However, this solution does not address the loss of nutrition and, as a result, cannot provide a sufficient kidney replacement.

In addition to chronic causes for kidney failure, acute causes of kidney failure are usually also treated with dialysis. In the case of acute kidney failure, the above shortcoming of dialysis also apply. In addition, the dialysis often does not address the reasons for the kidney failure but rather lessens the symptoms.

The objective of the present disclosure is to provide an improved approach to treating kidney failure. This objective is met by the medical system for supporting native kidney function of a patient's kidney according to claim 1. The dependent claims describe further developed embodiments of the medical system according to claim 1.

The medical system according to claim 1 comprises a fluid source for providing fluid to be injected into the patient's kidney, a fluid outlet in fluid connection with the fluid source, for transferring the fluid to a renal cortex of the patient's kidney, and a control unit that receives physiological condition information indicative of a physiological condition of the patient and that is configured to control a flow through the fluid outlet using the physiological condition information.

The medical system according to the present disclosure is based on the finding that the kidney can be supported in many cases of acute and also of chronic kidney failure by injecting fluid directly into the cortex of the patient's kidney. The fluid to be injected depends on the individual cause of the kidney failure. Independent of the fluid, the injection of fluid into the kidney is a challenging endeavour as a total amount of fluid or a rate of fluid that can be injected into the kidney depends highly on a condition of the kidney itself, but also on the condition of the patient's body. The medical system according to this disclosure provides a solution that typically enables a secure and continuous injection of fluid into the kidney. Central to that solution is the control unit that receives the physiological condition information and that is configured to control the flow through the fluid outlet in accordance with the physiological condition information and, as a result, control the flow to be adequate to the physiological condition of the kidney and the patient in general.

In the following, further developed embodiments of the medical system will be described. The description of the embodiments will begin with a description of embodiments comprising different sources for the provision of the fluid.

In one embodiment, the fluid source comprises a fluid reservoir for storing the fluid. This embodiment of the medical device is particularly advantageous in the case of an acute kidney failure. In case of an acute kidney failure due to loss of blood pressure, it is typically advantageous to flush the kidney with a fluid to provide the kidney with nutrition to keep the kidney alive. In case of an acute kidney failure due to infections, poisoning or inflammation, it is also advantageous to provide the kidney with additional fluid in order to clean out malicious substances or pathogens.

In a variant of this embodiment, the fluid to be stored in the fluid reservoir comprises purified isotonic solution. In another variant of this embodiment, the fluid source additionally or alternatively comprises an additive reservoir for storing an additive and is configured to add the stored additive to the fluid. In some variants, this additive is a medication. In other variants, the additive is additionally or alternatively a nutritional supplement relevant for the kidney. In yet another variant, the medical system comprises additionally or alternatively a heating unit for preheating the fluid received from the fluid source before the fluid is transferred through the fluid outlet.

In another variant, the medical system comprises a fluid-removal unit that is configured to remove fluid from a patient's blood, wherein the control unit is configured to control an amount of fluid that is removed from the patient's blood by the fluid-removal unit in accordance with the flow of fluid through the fluid outlet.

In another embodiment, the fluid source comprises a peritoneal extraction unit for extracting peritoneal fluid from a patient's peritoneal space, wherein the peritoneal extraction unit is configured to extract peritoneal fluid from a peritoneal inlet and to provide the extracted peritoneal fluid as the fluid to be injected into the patient's kidney. Furthermore, the medical system comprises a fluid-transfer unit for transferring fluid from the cortex of the patient's kidney to the patient's peritoneal space, wherein the fluid-transfer unit is configured to extract fluid through a renal inlet and provide that fluid at a peritoneal outlet. This embodiment is particularly advantageous to support peritoneal dialysis as it is commonly known. The idea of peritoneal dialysis is to use a patient's peritoneum as a filter membrane to filter the blood. One of the short-comings of peritoneal dialysis is that high-molecular-weight proteins cannot be handled adequately by the peritoneum. By using the embodiment of the medical system as described above, it is possible to transfer those high-molecular-weight molecules from the peritoneal space to the cortex of the kidney, where the molecules can typically be adequately metabolized by the microvilli seam of the tubular cells within the patient's kidney. Remaining fragments of the metabolized molecules can then be transferred back to the peritoneal space by the transfer unit or partially to the venous blood system within the kidney.

As the peritoneal fluid has a higher glucose concentration than the glucose concentration in the blood, the amount of fluid from the peritoneum injected into the kidney has to be monitored to ensure that the glucose concentration in the blood stays within safe boundaries. To this end, in one variant, the physiological condition information comprise a physiological parameter indicative of the glucose concentration in the blood and the control unit is configured to adjust an amount of fluid injected into the kidney using the physiological parameter. The physiological parameter may, for example, be the glucose concentration in the blood itself.

In addition, the injection of peritoneal fluid into the cortex might lead through absorption of the fluid into the blood to a loss of peritoneal fluid as well as to an increase of the patient's blood volume. Therefore, in variants, the physiological condition information, additionally or alternatively, comprise a physiological parameter indicative of a relative volume change regarding the peritoneal fluid and/or a relative volume change regarding the patient's blood volume and the control unit is configured to adjust an amount of fluid injected into the kidney using the physiological parameter. For the blood volume, such a parameter could be the haematocrit.

In yet another embodiment, the fluid source comprises a separation unit that is configured to separate primary urine from a patient's blood received through a blood inlet and to output the primary urine through a primary-urine outlet for transferring the primary urine to a renal cortex of the patient's kidney with the intention to perform dialysis.

The embodiment of the medical system as described above is based on the finding that many diseases that lead to kidney failure are primarily glomerular diseases that affect the filtration function of glomeruli comprised within the patient's kidney. The remaining part of the patient's kidney, in particular the tubules and collection ducts (including Henle's loop), in many cases, is not affected by those diseases, in particular because the deeper regions of the kidney have a separated vascular system. Therefore, the medical system according to the present disclosure aims at technologically replacing the glomerular function and providing primary urine at the right place - the cortex - thus allowing to make use of the remaining and still functional part of the patient's kidney, in particular the tubules and collections ducts.

A human kidney is made up of about a million nephrons, including the glomeruli that are essentially the filter units of the kidney. In the nephrons, urine generation is essentially performed in a two-step process. Blood is received through the renal artery and passed on to the glomerulus. In a first step, specialised cells within the glomerulus allow smaller molecules, wastes, and fluid (plasma water) to pass into the tubule attached to the glomerulus. Larger ingredients, such as proteins and blood cells, stay in the blood vessel. In a second step, as the filtered fluid moves along the proximal tubule, blood vessels running close to the tubules reabsorb almost all of the water, along with minerals and nutrients. Fluid and waste remaining in the tubule are up-concentrated and transported down to the bladder becoming secondary urine. Filtered blood is returned to the patient's body through a renal vein, essentially within the cortex.

The separation unit of the medical system is configured to take over the first of the two functions of the glomeruli which are to filter and also to administer the filtrate to the tubules. The system separates primary urine, which is comparable to filtrate produced by the glomeruli that is received by the tubules, from the patient's blood. The artificial primary urine can then be introduced into the cortex region within the patient's kidney through the primary-urine outlet. The tubules and collection ducts can then be used to up-concentrate the artificial primary urine. Experiments have shown that the paracellular introduction of primary urine from interstitial tissue into the tubules is possible. In particular, a proximal part of the tubules' wall is normally sufficiently open to let fluid enter from interstitial space. So interstitial fluid with the same or slightly higher pressure than the tubules' interior pressure will enter at least to a relevant extent into the tubules. Thus, by injecting the primary urine into the renal cortex of the patient's kidney under convenient pressure, the primary urine will typically find its way into the tubules, where the nephron can perform its normal function of up-concentrating the artificial primary urine as if it was native.

The described approach differs from prior art hemodialysis approaches in that the primary urine - its analogue is the hemodiafiltrate - has to be introduced into the tubules of the kidney instead of being replaced and discarded. The tubules sufficient vitality and functionality is an essential precondition of the applicability of the invention. This is fulfilled in many but not all cases if the onset of the method is early enough in the progress of chronic kidney disease (CKD). To ensure that the patient is not harmed and the kidney is not damaged in the process, physiological conditions of the patient's body have to be monitored and the flow and pressure of primary urine into the kidney has to be controlled accordingly. This task is performed by the control unit that receives physiological condition information indicative of a physiological condition of the patient and is configured to control the flow of primary urine through the primary-urine outlet into the cortex accordingly.

The approach of treating kidney diseases described above is usually advantageous, because it only requires a technical solution to replace the functions of the glomeruli while the tubules of the patient's kidney are still being used. As a result, a bioreactor comprising renal cell cultures that take part in the filtering of the blood is not required. Thus, some embodiments of the medical system do not comprise a bioreactor with renal cell cultures.

In a variant of this embodiment, the separation unit comprises, additionally or alternatively, a filter unit for separating the primary urine from the blood through hemofiltration. Hemofiltration is primarily based on filtering the received blood through convection. Using hemofiltration is typically advantageous, as it does not require a dialysate. As a result, the separation unit is implantable into the patient's body to provide a mobile dialysis. In case of hemofiltration, the primary urine is also referred to as ultrafiltrate. In a variant of this embodiment, the filter unit is at least partly made out of a semiconductor material. Semiconductor material based filters are typically very durable.

In another variant of this embodiment, the medical system comprises, additionally or alternatively, an additive storage unit for storing one or more additives. Furthermore, the medical system is configured to add a portion of the one or more additives to the primary urine before the primary urine is transferred to the renal cortex of the patient's kidney. Additives can be used to enrich the primary urine with, for example, nutrients, buffers, electrolytes, oxygen, medication, water or any combination thereof. In other embodiments, the medical device is, additionally or alternatively, configured to add portions of the additives to the patient's blood that is to the filtered blood or to the blood before it passes through the separation unit.

In another variant, the medical system comprises a renal extraction unit for extracting renal fluid from a patient's kidney, wherein the renal extraction unit is configured to extract renal fluid via an extraction inlet. The extraction inlet can be an additional inlet or the fluid inlet used bi-directionally. This embodiment is typically useful in those cases, when an excess amount of primary urine resides in the patient's kidney, for example, because the kidney did not process the primary urine as expected. In this case, the extraction unit can be used to remove primary urine from the kidney to lower a fluid pressure inside the kidney or to maintain a continuous flow through the kidney in cases the tubules and/or the veins do not or do not sufficiently transport the primary urine. This embodiment can typically also be used in another scenario, in which the tubules are not used to perform the second step of the two-step filter process, for example, because the tubules are not fully functional. In this case, it can still be of value to bring the primary urine in contact with an internal cell surface of the proximal tubules cells, enabling support of metabolism of middle and high molecular weight uremic toxins in particular or for nutritional purposes of the kidney tissue, but not exclusively to support regular dialysis. The primary urine is then transferred into the kidney and then removed again. In one realization, the renal extraction unit is configured to provide the extracted renal fluid at an outlet that is connectable to a vessel of a patient's native vascular system for discharging it into the patient's blood stream.

In another variant, the medical system comprises a housing that includes at least the separation unit and the control unit, wherein the housing is adapted to be implanted into the patient's body to provide mobile dialysis.

The physiological condition information can comprise a plurality of information. In one embodiment, the physiological condition information comprises primarily information about a condition of the patient's kidney. In one variant of this embodiment, the physiological condition information comprises information indicative of a pressure within the kidney. In another variant, the physiological condition information includes, additionally or alternatively, information indicative of a fluid leaking from the kidney. In other embodiments, the physiological condition information additionally or alternatively comprises information regarding the patient's body in more general terms. In one variant of this embodiment, for example, the physiological condition information comprises, additionally or alternatively, information regarding a patient's blood circuit. In another variant of this embodiment, the physiological condition information comprises, for example, information about absolute or relative blood volume. In one of these variants, the control unit is configured to reduce and/or to stop the flow of fluid through the fluid outlet, when the relative blood volume drops below 90 % or below 80%. In yet another variant, the physiological condition information comprises, additionally or alternatively, information about a patient's heart rate, a patient's rate of breath, a patient's blood pressure, a patient's body weight and/or an electrocardiogram. In another variant, the physiological condition information comprises, additionally or alternatively, information regarding haematocrit. In realizations of this variant, the control unit is additionally or alternatively configured to reduce and/or to stop the flow of fluid through the fluid outlet, when the haematocrit exceeds a maximum level, for example ranging from 55 to 58%. In one variant, the physiological condition information comprises, additionally or alternatively, information about a patient's body temperature, and/or a body-fat-ratio or a body-composition ratio of the patient's body.

With regard to controlling the flow of the fluid through the fluid outlet, the control unit of one embodiment is configured to regulate, using the physiological condition information, an amount of fluid that is output through the fluid outlet per unit time. In another embodiment, the control unit is, additionally or alternatively, configured to regulate a pressure of the fluid at the fluid outlet. In particular, some embodiments are configured to provide the fluid with a minimal pressure for the fluid to enter the tubules. Other embodiments are, additionally or alternatively, configured to provide the fluid with a maximal pressure so as to not damage the patient's kidney. In one variant of these embodiments of the medical system, the medical system is configured to provide the fluid at the fluid outlet with a pressure in a range between 60 and 100 mmHg, in particular between 70 and 100 mmHg. This pressure is typically particularly suited for inserting fluid into the cortex of the patient's kidney.

Some embodiments of the medical system, additionally or alternatively, comprise one or more fluid conduit units with a connection end that is connected to the fluid outlet and an output end that is insertable into the renal cortex of the patient's kidney for discharging the fluid into the renal cortex of the patient's kidney. The fluid conduit unit of some of those embodiments comprises one or more cannulas. Those cannulas can be designed to branch off from each other inside or outside of the kidney. In other embodiments, the fluid conduit unit comprises, additionally or alternatively, a securing member that is, when the output end is inserted into the renal cortex, configured to hold the output end in place and/or reduce leakage of fluid from the patient's kidney surrounding the output end. In variants of this embodiment, the securing means is configured to altogether avoid leakage of fluid from the patient's kidney. In other variants of this embodiment, the securing member, additionally or alternatively, comprises barbs for holding the output end in place. In yet other variants, the securing member, additionally or alternatively, comprises inflatable cuffs and/or an aperture to receive suture. The inflatable cuffs can be advantageous for holding the output end in place and reducing leakage. The inflatable cuffs, in one variant, comprise one or more inflatable sealing elements that are configured to be inflated after the fluid conduit unit is inserted into the cortex to provide a seal for fluid.

In some embodiments of the medical system that comprise a fluid conduit unit, the fluid conduit unit comprises additionally or alternatively a penetration-indication unit that is configured to detect when the cannula penetrates the capsule. In some realizations, the penetration-indication unit comprises an optical detection unit that is configured to optically detect a penetration of the capsule using an optical wave guide, and a light source. The wave guide is arranged within the cannula such that one end of the wave guide is positioned in proximity to an opening end of the cannula which is to be inserted into the kidney. Moreover, the penetration-indication unit is configured to couple light emitted by the light source into the wave guide and to guide back-scattered light that is back-scattered at the one end to the optical detection unit. Furthermore, the optical detection unit is configured to detect a difference in relative phase and/or intensity of the back-scattered light indicative of a change in refractive index of the medium surrounding the one end of the wave guide. Due to the difference in refractive index of the capsule and the cortex, it is possible to detect whether the opening end of the cannula is located within the cortex or the capsule. The fluid conduit unit comprising the penetration-indication unit may help a medical professional to insert the cannula into the cortex directly beneath the capsule, that is at a depth of up to 10 mm from an outer side of the capsule for an adult. Limiting the depth of insertion typically reduces a risk of injury of the kidney.

In yet another variant of the embodiment, the medical device comprises, additionally or alternatively, a sealing member that is configured to at least partly enclose a renal capsule of the patient's kidney to prevent an escape of fluid from a puncture of the renal capsule. In one realization of this variant, the sealing member comprises a sterile enclosure bag. The tension of this bag can also be measured to analyze modifications of the kidneys capsule compliance. In a realization of such a variant, the sealing member is made of one or more chemical substances, in particular proteins or chain-like molecules, that are directly applied to the capsule of the patient's kidney to prevent escape of fluid from pores of the capsule.

In another embodiment, the medical system comprises, additionally or alternatively, a sensing unit that is configured to detect one or more measurement quantities of a patient's body indicative of a physiological condition of the patient's body and to provide the physiological condition information to the control unit. In variants of this embodiment, the sensing unit is configured to measure vital signs of the patient, for example pulse, blood pressure and/or oxygen saturation of the blood. In other variants, the sensing unit is, additionally or alternatively, configured to perform (relative) volumetric measurements, for example of the blood, haematocrit measurements, temperature measurements, blood viscosity measurements, plasma conductivity and/or urine conductivity or uremic toxin content in the respective fluids or urine flow rates. In other variants, the sensing unit comprises, alternatively or additionally, a fluid detection unit for detecting fluid, in particular primary urine leaking from the patient's kidney. In another variant, the sensing unit comprises, additionally or alternatively, a strain detection unit that is configured to detect excess strain of the capsule, for example, through determining an electrical conductance of the renal capsule or fluid on its surface. In another variant, the sensing unit is additionally or alternatively implantable into the patient's body. In another variant, the sensing unit comprises an ultrasound scanner for, for example, determining a leakage of fluid from the kidney.

In a further embodiment, the medical system, in particular the separation unit and/or the fluid-conduit unit, is, additionally or alternatively, implantable into a patient's body. This can further help to make the medical system restore the full functionality of the patient's kidney.

In another embodiment, the medical system comprises, additionally or alternatively, an electrical-energy provision unit that is configured to provide electrical energy to be used for operating the medical system. In variants of this embodiment, the electrical-energy provision unit is configured to source energy from a patient's body, for example by using an electrical microgenerator that is driven through a patient's blood pressure or through expanding or contracting blood vessels of the patient's. In another variant, the electrical-energy provision unit, additionally or alternatively, comprises an electrical-energy storage unit such as a (rechargeable) battery. In yet another variant, the electrical-energy provision unit is configured to source electrical energy from electromagnetic radiation or magnetic fields traversing the skin.

In another embodiment, the medical system, additionally or alternatively, comprises a fluid pump for transporting the fluid, blood and/or the primary urine. The term blood in this case can relate to the filtered blood and/or the blood received from the patient's body. In a variant of this embodiment, the fluid pump is configured to use a patient's blood pressure for transporting the fluid, blood and/or the primary urine.

In a different embodiment, the medical system, additionally or alternatively, comprises a bubble detector that is configured to detect gaseous bubbles in the fluid before it is discharged through the fluid outlet and, when gaseous bubbles are detected in the fluid and an amount of those gaseous bubbles surpass a pre-defined upper limit, stops the fluid from being output through the fluid outlet. In variants of this embodiment, the medical system comprises a degassing unit which is configured to degas the fluid. Introducing small amount of gaseous bubbles into the kidney is not dangerous to the patient, but lowers an efficiency of the approach. However, gaseous bubbles may lower the efficiency of a fluid transport within the patient's kidney. In addition, larger amounts of gaseous bubbles can pose a threat to the patient's health.

In another embodiment, the medical system, additionally or alternatively, comprises an ultrasound unit including an ultrasound probe head, wherein the ultrasound probe head is configured to be placed at the patient's kidney. Furthermore, the control unit is configured to activate the ultrasound unit such that the ultrasound unit emits ultrasound waves for widening narrow fluid paths within the kidney by sonic vibration, in particular at the proximal tubules. This may enhance the water transit at their junctions. In variants of this embodiment, the control unit is configured to activate the ultrasound unit depending on the physiological condition information.

In yet another embodiment, the control unit is configured to control the flow of fluid by adjusting an operating power of a pump and/or a width of an adjustable opening in accordance with the physiological condition information.

In the following, in addition to the medical device, a method for supporting native kidney function of a patient's kidney will be described.

The method comprises the following steps:
- receiving a fluid from a fluid source,
- injecting the fluid into a cortex of the patient's kidney.

The method typically has the same advantages as the medical system described above.

In the following, different embodiments of the method will be described.

In one embodiment, the fluid received from the fluid source comprises purified isotonic water.

In a variant of this embodiment, the method additionally comprises removing fluid from a patient's blood. In another variant of this embodiment, the method comprises pre-heating the fluid before injection into the patient's kidney.

In another embodiment, the fluid is injected into the cortex of the kidney directly beneath the capsule. For example, in one embodiment, the fluid is injected within a depth of up to 10 mm measured from an outside surface of the capsule.

In another embodiment, the fluid received from the fluid source is fluid extracted from a peritoneal space of the patient, wherein the method comprises the steps:
- removing fluid that is to be injected into the cortex from the peritoneal space of the patient, and
- transferring fluid from the patient's kidney to the peritoneal space.

The peritoneal fluid has a higher glucose concentration than the glucose concentration in the blood. As a result, the amount of fluid from the peritoneal injected into the kidney has to be monitored to ensure that the glucose concentration in the blood stays within save boundaries. As a result, in one variant, the embodiment comprises a step of monitoring a physiological parameter indicative of the glucose concentration in the blood and adjusting an amount of fluid injected into the kidney using the physiological parameter. The physiological parameter may, for example, be the glucose concentration in the blood itself.

In addition, the injection of peritoneal fluid into the cortex might lead through absorption of the fluid into the blood to a loss of peritoneal fluid as well as to an increase of the patient's blood volume. Therefore, in variants, the method additionally or alternatively comprises a step of monitoring a physiological parameter indicative of a relative volume change regarding the peritoneal fluid and/or a relative volume change regarding the patient's blood volume. For the blood volume, such a parameter could be the haematocrit.

In a variant, injection of fluid into the cortex and extraction of fluid from the cortex takes place at different areas of the cortex such that injected fluid is not immediately removed but rather remains in the kidney for a certain time span. The same can be done for the injection of fluid into and extraction of fluid from the peritoneal space. In other variants of this embodiment, the method additionally or alternatively comprises performing or supporting a peritoneal dialysis on the patient while the method is performed.

In yet another embodiment, the fluid received from the fluid source is primary urine and the method comprises the further steps:
- drawing blood from the patient, and
- separating primary urine from the blood drawn from the patient for injection into the cortex of the patient's kidney.

In a variant of this embodiment, the method comprises extracting fluid from the kidney. This means, the fluid filtered from the blood and once injected before into the cortex is at least partly extracted again from the kidney after a certain time. This can be advantageous to augment or support traditional dialysis by enhancing contact time of primary urine with the microvilli seam of the proximal tubule cells and further to reduce pressure in the patient's kidney if necessary in certain situations. In certain cases, the patient's kidney is not able to produce secondary urine from the injected primary urine. This can, for example, be the case when the tubules of the kidney are not fully functional any more or are obstructed by preceding disease. However, it can still be advantageous to bring the kidney in contact with the primary urine, in parallel to conventional hemodialysis or peritoneal dialysis. The contact of the primary urine with an internal cell surface of the proximal tubules cells, typically enables support of metabolism of middle and high molecular weight uremic toxins and metabolites - a functionality conventional dialysis is fairly ineffectual with. The method in this variant can be utilized as a support of conventional dialysis.

In another variant of this method, the fluid is, additionally or alternatively, extracted from other parts of the body and not from the blood, for example, to extract fluid that leaked from the kidney.

In one embodiment of the method, the method comprises additionally or alternatively the following steps:
receiving physiological condition information indicative of a condition of the patient's body, and
controlling a flow of the fluid injected into the renal cortex in accordance to the physiological condition information.

In other embodiments of the method, the fluid is, alternatively or additionally, inserted into the renal cortex through an entrance in the capsule of the patient's kidney. In another embodiment, the primary urine is inserted into the renal cortex through a patient's pelvis and/or along a vessel, such as an artery or a vein by leaving the vessel with the injection device at the desired site within the cortex.

In all of the methods described above that include the extraction of fluid from the patient's body, the fluid can be discharged into the patient's blood stream.

In the following, the medical system and the method according to the present disclosure will be further described with the help of the figures. At first, an overview of the Figures will be provided.
- Fig. 1: shows a medical system for supporting native kidney function of a patient's kidney;
- Fig. 2a: shows a cross-sectional view of a section of a patient's kidney during treatment with the medical system shown in Fig. 1;
- Fig. 2b: shows an enlargement of the cross-sectional view as shown in Fig. 2a focussing on a nephron;
- Fig. 3a: shows a medical system comprising a primary-urine transfer unit and a sensing unit;
- Fig. 3b: shows a primary-urine conduit unit comprising a penetration-indication unit;
- Fig. 4: shows a sealing member for use with the medical systems of Fig. 1 and Fig. 3a;
- Fig. 5: shows a medical system that comprises a fluid extraction unit;
- Fig. 6: shows a medical system that comprises an ultrasound unit;
- Fig. 7: shows a medical system that uses a fluid reservoir for sourcing the fluid to be injected into the patient's kidney;
- Fig. 8: shows a medical system comprising a fluid-removal unit;
- Fig. 9: shows a medical system for supporting peritoneal dialysis;
- Fig. 10: shows an example for a method for supporting native kidney function of a patient's kidney;
- Fig. 11: shows a method for supporting native kidney function of a patient's kidney wherein a fluid is received from a fluid reservoir;
- Fig. 12: shows a method used in support of a peritoneal dialysis;
- Fig. 13: shows a method for supporting native kidney function of a patient's kidney, wherein primary urine is injected into the kidney; and
- Fig. 14: shows a method that comprises extracting fluid from the patient's body.

In the following, a detailed description of the embodiments shown in the Figures will be provided. In all the Figures of the present disclosure, identical reference signs will be used to describe identical entities. For brevity, description of a Figure will mainly focus on describing those entities that have not appeared in an earlier Figure.

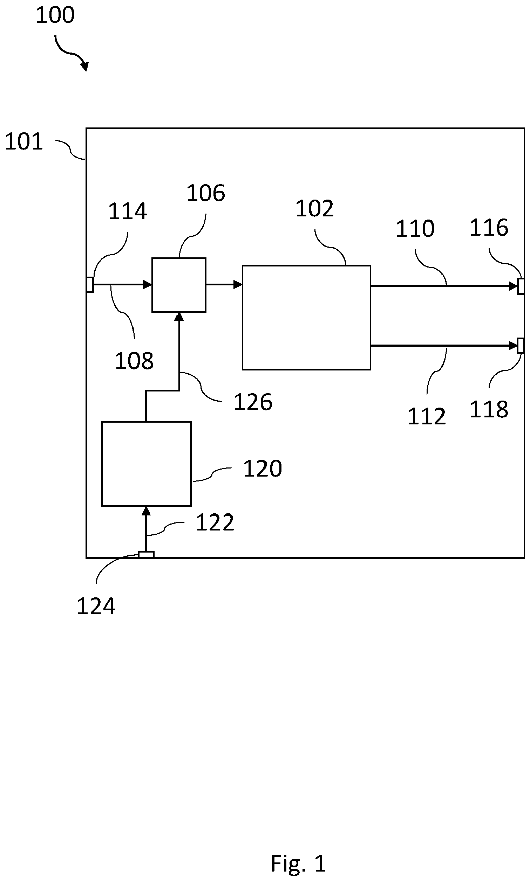

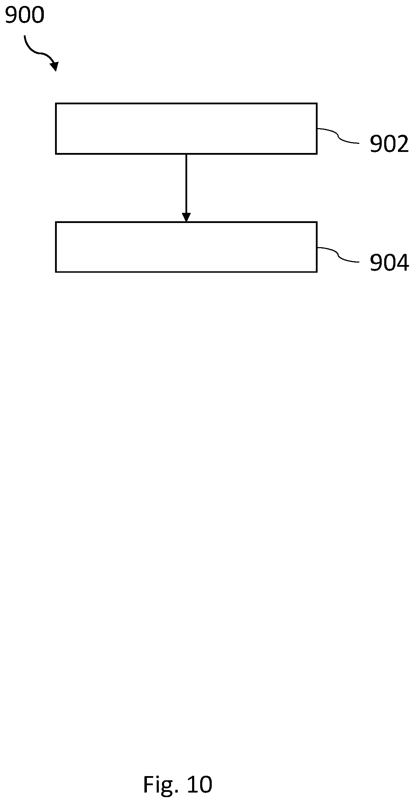

At first, a description of different medical devices for supporting a patient's native kidney function will be provided with reference to Figs. 1-9. Later related methods for supporting a patient's native kidney function will be described with reference to Figs. 10-14.

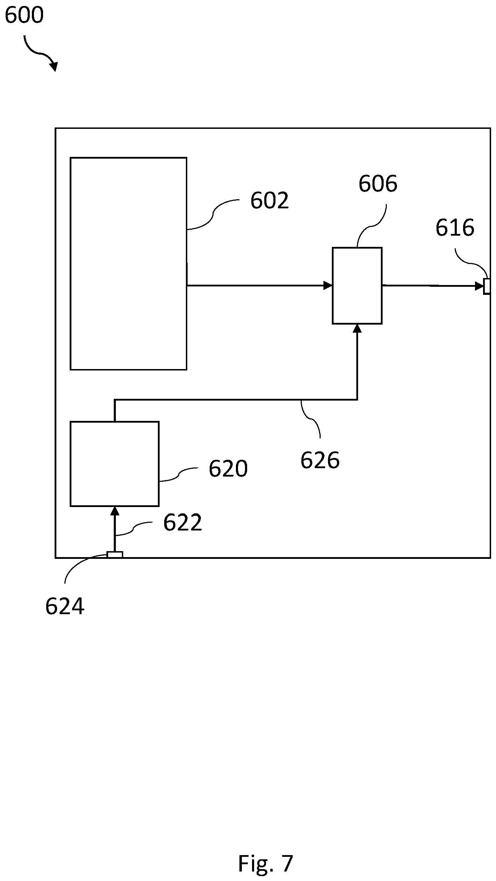

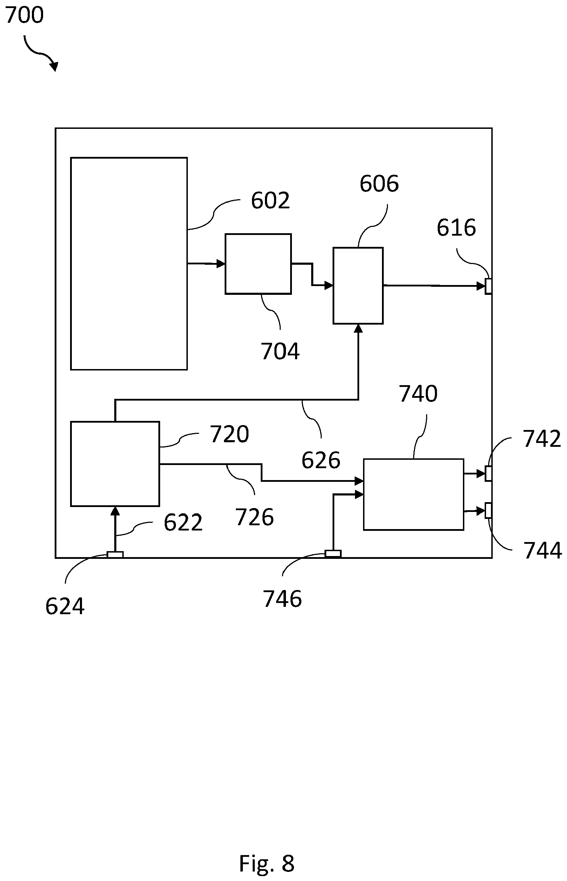

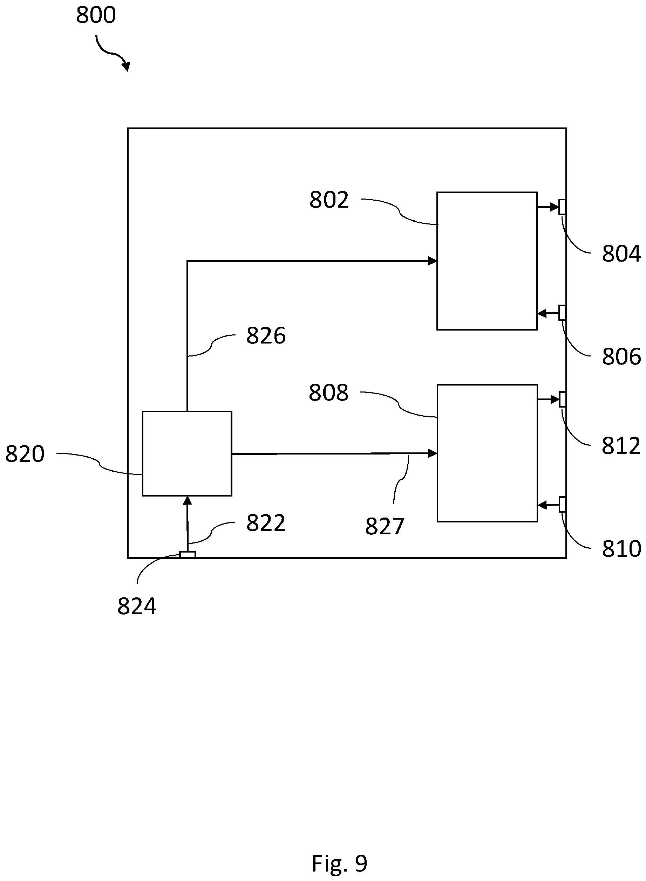

The medical devices shown in Figs. 1-9 comprise various fluid sources. The description will start with the medical devices shown in Figs. 1-6, which all comprise a separation unit for separating primary urine from a patient's blood as the fluid source. Afterwards, the medical devices shown in Fig. 7 and Fig. 8 will be described that comprise a fluid reservoir. Lastly, the medical device shown in Fig. 9 will be described that comprises a peritoneal extraction unit as the fluid source.

Fig. 1 shows a medical system 100 for supporting native kidney function of a patient's kidney.

The medical system 100 includes as a fluid source separation unit 102 that is configured to separate primary urine 112 from a patient's blood 108 received through a blood inlet 114 and to output the primary urine through a fluid outlet referred to as the primary-urine outlet 118 for transferring the primary urine 112 to a renal cortex of the patient's kidney.

Furthermore, the medical system 100 comprises a control unit 120 that receives physiological condition information 122 indicative of a physiological condition of the patient through a condition-information input interface 124 and is configured to control a flow of the primary urine through the primary-urine outlet 118 using the physiological condition information.

Through the medical system 100 shown in Fig. 1, the native kidney function of a patient with a glomerular disease can be supported. The separation unit 102 receives the patient's blood 108 through the blood inlet 114 and is configured to separate primary urine 112 from the blood 108 and, as a result, output filtered blood 110 at a blood outlet 116 and primary urine 112 at the primary-urine outlet 118. The filtered blood can be returned to the patient by, for example, introducing the filtered blood 110 into a patient's vein. The primary urine 112 can be inserted into a renal cortex of the patient's kidney, where the primary urine can be taken up by tubules and veins of the kidney.

The separation of blood 108 into primary urine 112 and filtered blood 110 is performed through a filter unit (not shown) that is part of the separation unit 102. The filter is used to separate primary urine from the blood using a membrane, which in the case of medical device 100 composes a semiconductor material into which slits are engraved, dimensioned to mimic primary filter slits of podocytes present within the glomerulus. However, any other membrane that is able to separate the primary urine from the patient's blood can also be used. For simplicity, the term "filtered blood" is used above to describe the blood from which the primary urine is removed, even though the blood itself does not pass the filter. Furthermore, the term "primary urine" is used to describe the fluid including the substance that are removed from the blood by passing through the filter.

In the example shown in Fig. 1, the medical system 100 uses a patient's blood pressure to pump the patient's blood through the separation unit. However, as will be explained later, it is also possible to instead use one or more fluid pumps if a patient's blood pressure is not sufficient.

The control unit 120 is configured to generate a fluid control signal 126 using the physiological condition information 122. The medical device 100 comprises a flow control unit 106, which receives the fluid control signal 126 and is configured to control a flow of the patient's blood 108 through the separation unit 102 and in turn also the flow of the primary urine 112 through the primary-urine outlet 118. The flow control unit 106 is configured to control a flow of the blood by reducing or increasing a cross section of a fluid channel through which the blood passes. An additional flow control unit can also be placed upstream from the separation unit 102 between separation unit 102 and primary urine outlet 118.

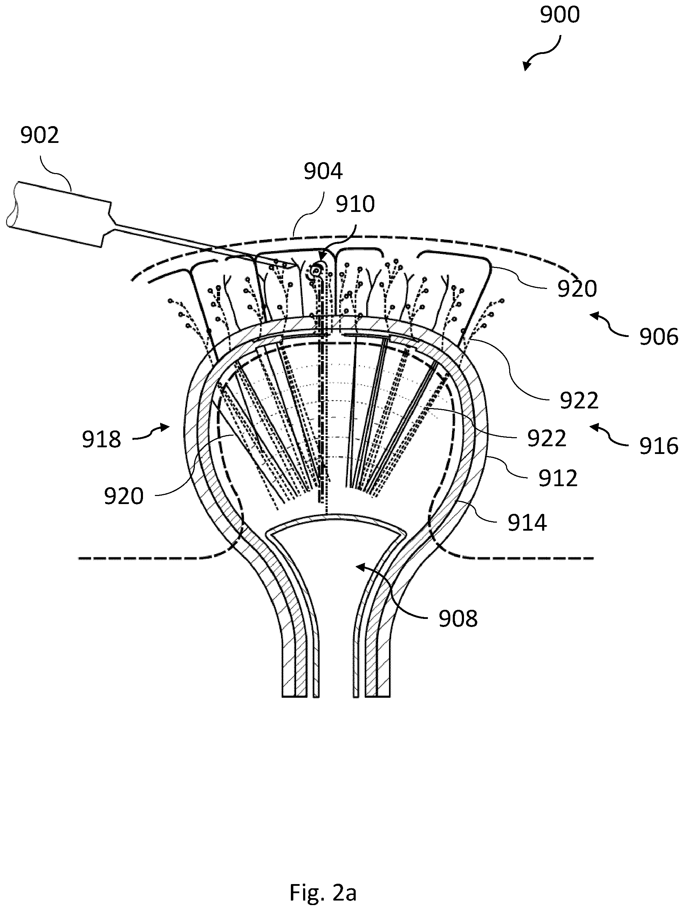

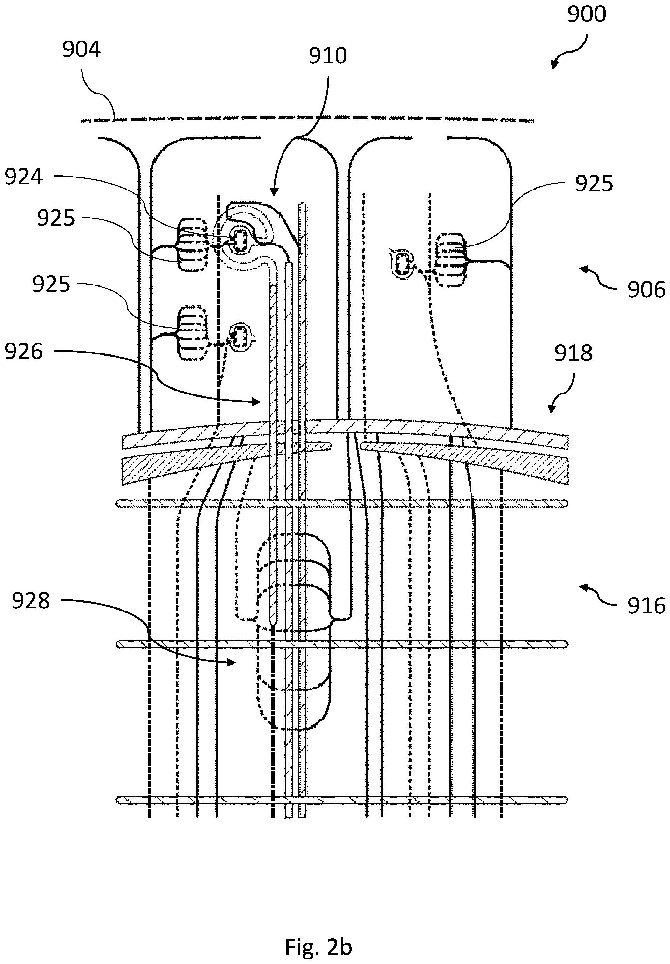

How the medical device 100 can be used to support native kidney function will be explained with regard to Fig. 2a and Fig. 2b.

Fig. 2a shows a cross-sectional view of a section of a patient's kidney 900 during treatment with the medical system shown in Fig. 1. Fig. 2b shows an enlargement of the cross-sectional view as shown in Fig. 2a focussing on a nephron 910.

The kidney 900 shows a renal capsule 904, which forms an outer layer of the kidney 900. Below the renal capsule 904, areas of the kidney referred to as renal cortex 906 and renal medulla 916 are indicated. The renal cortex 906 and the renal medulla 916 are shown in Fig. 2a in the vicinity of a renal pyramid 918. The renal pyramid 918 is surrounded by an artery 914 that branches off into smaller vessels 922 indicated by dashed lines inside the renal cortex 906 and the pyramid 918. Furthermore, the renal pyramid 918 is also surrounded by a vein 912 that also branches of into smaller vessels 920 inside the renal cortex 906 and the renal pyramid 918. Furthermore, Fig. 2a indicates in an abstract form a renal nephron 910, which is illustrated in more detail in Fig. 2b.

Nephrons, such as the nephron 910 are essential for the purification of the patient's blood within the kidney 900. To this end, the nephron 910 comprises at a proximal part a glomerulus 924. In a first step of the purification process, thin slit walls within the glomerulus allow smaller molecules, wastes, and fluid to pass into the tubule 926 surrounding the glomerulus with its proximal part. Larger molecules, such as proteins and blood cells, stay in the blood vessel. In a second step, as the filtered fluid moves along the tubule 926, blood vessels 928 running along the tubule 926 almost all of the water, along with minerals and nutrients is reabsorbed. Remaining fluid and wastes in the tubule 926 are collected from all nephrons that are part of the renal pyramid 918 attached to a collective duct and finally to a vessel referred to as calix 908, from where the collected fluid, the urine, is transported to the bladder.

In case that the glomeruli of the kidney 900 are not functional or only show a limited functionality due to, for example, a disease, the medical system 100 can be used to support the native kidney function. To this end, the patient's blood is diverted towards the medical system 100, where it is filtered with the help of the separation unit 102. The resulting primary urine 112 is then injected into interstitial space within the renal cortex 906, for example, with a cannula 902 as shown in Fig. 2a. From there the primary urine 112 can be transported by convenient pressure into the proximal part of the tubule 926 as well as into venous vessels 920. A small fraction of the primary urine moves along the tubule 926. As in a healthy kidney it is up-concentrated like native primary urine and finally collected from all nephrons within the calix 908, from where secondary urine is transported to the bladder.

In contrast to common dialysis practice, where a patient would have to come to a special dialysis facility multiple times a week, the medical system 100 is suitable for providing continuous filtering of the blood to those patients eligible due to still open tubules, in particular because no dialysate is required and the excess fluid not ending up as secondary urine returns to the body via the capillary systems 920 and peritubular capillaries 925 like native primary urine and therefore is not lost.

Through injection into the cortex of the patient's kidney, the primary urine can reach large parts of the kidney. This principle can also be used with regard to other fluids, which will be described in greater detail later.

The medical system 100 comprises a housing 101, which is adapted to be implanted into a patient's body to provide a truly mobile dialysis solution for the patient. However, it is also possible to operate the medical system 100 as an external device.

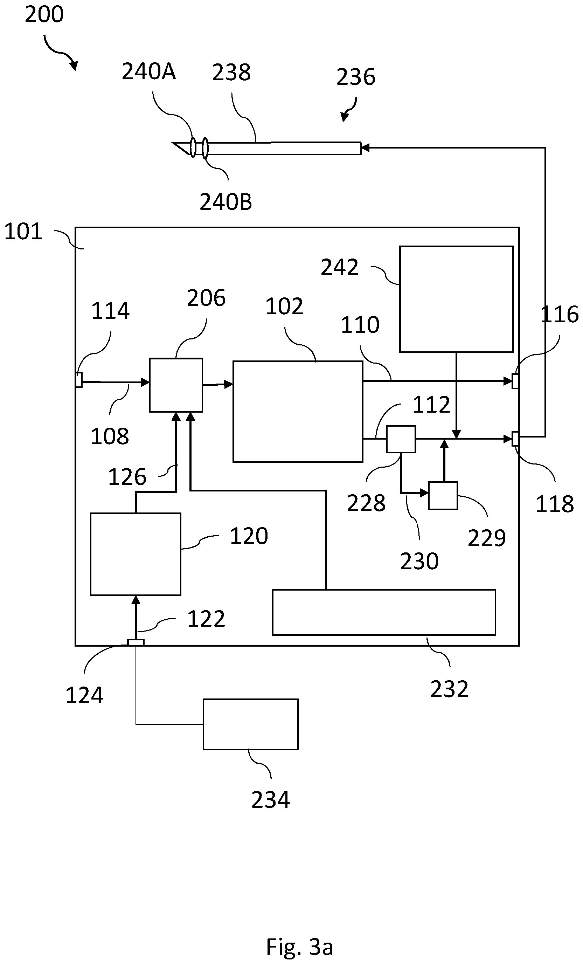

The medical system 100 shown in Fig. 1 does not show how the physiological condition information 122 is collected and details of how the primary urine 112 is transferred to the patient's kidney. Those two aspects will be described in more detail in the following with reference to Fig. 3a.

Fig. 3a shows a medical system 200 comprising a primary-urine conduit unit 236 and a sensing unit 234.

The medical system 200 comprises a primary-urine conduit unit 236 that is in fluid communication with the primary-urine outlet 118. The primary-urine conduit unit 236 comprises at an output end of the primary-urine conduit unit 236 a cannula 238 that is configured to be inserted into the renal cortex to transfer and release the primary urine 112 into the renal cortex of the patient's kidney. Furthermore, the primary-urine conduit unit 236 comprises securing means 240A and 240B that are, when the cannula 238 is inserted into the renal cortex, configured to hold the cannula 238 in place and reduce leakage from around a place of insertion of the cannula 238 into the patient's kidney. The securing means 240A and 240B are inflatable cuffs that are each formed an inflatable ring lining the circumference of the cannula 238. The cannula 238 is inserted into the patient's kidney such that securing means 240A enters the kidney and securing means 240B remains outside the kidney. Then the securing means 240A and 240B are inflated. The securing means 240A anchors the cannula 238 in the kidney while the securing means 240A and 240B, together, form a seal for fluid, in particular the primary urine, to prevent or at least reduce leakage from the kidney.

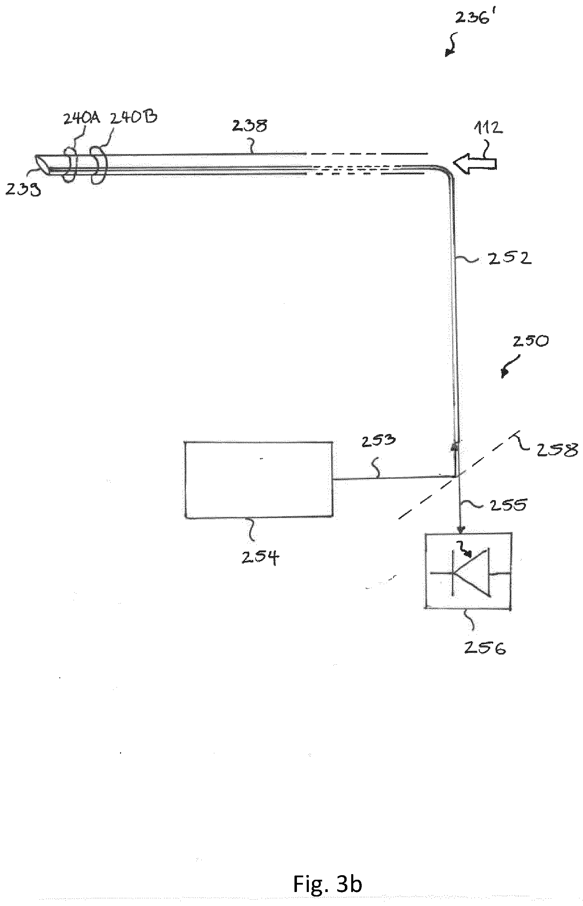

To reduce a risk to injure the kidney, it is typically advantageous to inject the primary urine in an area of the cortex that lies directly beneath the capsule. In some embodiments of the primary-urine conduit unit, the primary-urine conduit unit comprises a penetration-indication unit that is configured to detect when the cannula has penetrated the capsule and enters the cortex. Such an embodiment of the primary-urine conduit unit is shown in Fig. 3b, which will be described in more detail in the following.

Fig. 3b shows a primary-urine conduit unit 236' comprising a penetration-indication unit 250.

The primary-urine conduit unit 236' comprises the cannula 238 and the inflatable cuffs 240A and 240B already described with reference to Fig. 3a. In addition, the primary-urine conduit unit 236' comprises the penetration-indication unit 250. The penetration-indication unit 250 includes an optical fibre 252, a laser 254, a photodetector 256, and a semi-transparent mirror 258. The optical fibre 252 is arranged within the cannula such that one end of the optical fibre 252 is arranged in proximity to an opening end 239 of the cannula 238 which is to be inserted into the kidney. To detect when the opening end 239 of the cannula 238 penetrated the capsule and reached the cortex, a laser beam 253 generated by the laser 254 is coupled through the semi-transparent mirror 258 into the optical fibre 252 at another opposite end. In addition, back-scattering 255 of the laser beam 253 is measured by the photodetector 256. Due to the difference in refractive index between the capsule and the cortex of the kidney, from an analysis of the intensity as well as an analysis of a relative phase of the back-scattering 255 it can be determined whether the opening 239 of the cannula 238 has penetrated the capsule. The primary-urine conduit unit 236' is configured such that the penetration-indication unit 250 can be removed from the cannula 238 after the cannula has been inserted into the kidney.

In certain circumstances, the primary urine 112 can develop gaseous bubbles. Introducing small amounts of gaseous bubbles into the patient's kidney is not dangerous for the patient as small gas amounts are solved by aqueous fluids. However, the gaseous bubbles are likely to lower an efficiency of the fluid transport within the kidney. To this end, the medical system 200 comprises a bubble detector 228 that is configured to detect gaseous bubbles in the primary urine 112 and, when the bubble detector 228 detects gaseous bubbles, to reroute that primary urine 230 through a degassing unit 229.

The described approach to support the kidney does typically not entail a continuous supply of additives to the primary urine 112, as primary urine is not discarded but returned to the patient's body to be reused. Nevertheless, in certain circumstances it can be desirable to add, for example, further nutrition or oxygen to primary urine 112. For that reason, the medical system 200 comprises an additive storage unit 242 that is configured to store additives and to release portions of the additives into the primary urine 112 before it is transferred to the primary urine outlet 118.

The medical system 200 further comprises a sensing unit 234 that is electrically connected to the condition-information input interface 124. The sensing unit 234 is configured to detect a plurality of measurement quantities that are indicative of a physiological condition of the patient's body and, using those measurement quantities, generate the physiological condition information 122 and provide the physiological condition information 122 to the control unit 120. To this end, the sensing units comprises a strain sensor that can be attached to a renal capsule of the patient's kidney. The strain sensor is configured to detect a stretching of the capsule. By relaying the information about stretching of the capsule in the physiological condition information 122, the control unit 120 can adjust the pressure and/or flux of the primary urine 112 released from the medical system 200 into the patient's kidney. The strain sensor detects strain directly by force measurement or by measuring a conductivity of the capsule of the patient's kidney. Investigations have shown that the conductivity of the capsule is a reliable indicator of excess internal pressure within the kidney as small amounts of water transit nano-pores of the capsule when overstretched.

In contrast to the medical device 100, the medical device 200 does not comprise a flow control unit 106. Instead, the medical device 200 comprises a fluid pump assembly 206 comprising a fluid pump that are configured to draw the patient's blood through the blood inlet 114 and pump the blood through the separation unit 102. The pump assembly 206 receives the fluid control signal 126 and is configured, depending on the fluid control signal 126, to adjust a flow of the patient's blood 108 through the separation unit 102 and in turn also the flow of the primary urine 110 through the primary-urine outlet 116 by controlling the fluid pump. An additional fluid pump assembly can also be placed upstream from the separation unit 102 between separation unit 102 and primary urine outlet 118.

Furthermore, the medical system 200 comprises an electrical-energy provision unit 232, which is configured to supply electrical energy to the components of the medical system 200, in particular the fluid pump assembly. In the medical system 200, the electrical-energy provision unit 232 comprises an electrical-energy storage unit and a reception circuit (both not shown). The reception circuit is configured to source electrical energy from electromagnetic radiation as generated, for example, by a wireless charging device and store the received electrical energy in the electrical-energy storage unit. The use of a reception circuit is advantageous to make the medical system implantable into the patient's body. However, other embodiments of the medical system, the electrical-energy provision unit comprises an electrical-energy generating unit that is configured to generate electrical energy from the patient's blood pressure or, additionally or alternatively, generate electrical energy from a conversion of chemical energy.

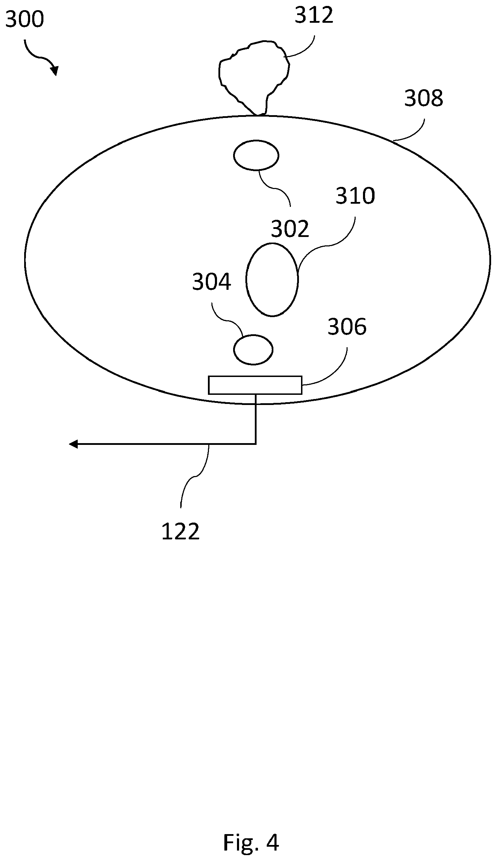

In other embodiments of the medical system, other kinds of sensing units and other primary-urine transfer means are used. An example of a combination of a sensing unit and an additional component for the primary-urine conduit means will be described in the following with reference to Fig. 4.

Fig. 4 shows a sealing member 300 for use with the medical systems of Fig. 1 and Fig. 3a.

The sealing member 300 comprises a sealing bag 308 that is configured to at least partially enclose the patient's kidney to prevent primary urine inserted into the kidney to leak from the kidney into the retroperitoneal space. To this end, the sealing bag 308 can be wrapped around the kidney forming a fluid tight bag with a vessel entrance.

The sealing bag 308 comprises two locks 302 and 304. Through lock 302, the cannula 238 of the primary-urine transfer means 236 can be inserted into an interior of the sealing bag 308 and inserted into the patient's kidney. Through lock 304, a fluid pipe can be inserted to withdraw excess fluid from either the sealing bag 308 or the kidney itself. Furthermore, the sealing bag 308 comprises a lock 310 through which arteries and veins as well as the ureter can be led. In addition, the sealing bag 308 comprises a leash 312 with which the sealing bag 308 and the patient's kidney within the sealing bag 308 can be secured inside the patient's body.

Furthermore, in the interior of the sealing bag 308, a sensing unit 306 comprising a fluid detector is located. The fluid detector is configured to detect primary urine leaking from the patient's kidney. When fluid leaks from the kidney and collects in the sealing bag 308, the sensing unit 306 is configured to detect the fluid and to relay the information in the form of physiological condition information 122 to the control unit 120.

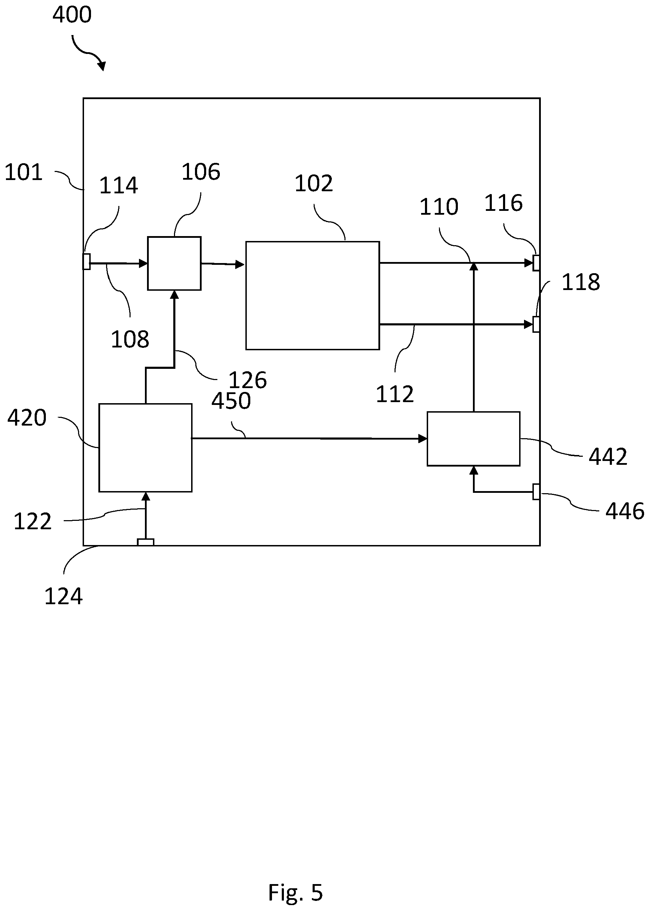

To extract fluid from the patient's kidney or the sealing bag 308, the medical system needs to be adapted accordingly. A medical system that is particularly suited for this purpose will be described in the following with reference to Fig. 5.

Fig. 5 shows a medical system 400 that comprises a renal extraction unit 442.

The renal extraction unit 442 receives an extraction control signal 450 and is configured, in dependence on the extraction control signal 450, to suck in fluid through a fluid inlet 446 with an additional fluid pump comprised within the fluid extraction unit 442 and to discharge the extracted fluid through the blood outlet 116. To this end, the medical system 400 comprises a control unit 420 that receives physiological condition information 122 and is configured to generate, using the physiological condition information 122, the extraction control signal 450. The fluid extraction unit 442 can be advantageous is those cases, where a fluid pressure within the patient's kidney is too high. Alternatively, the fluid extraction unit can be used to help augment traditional dialysis. In certain cases, the patient's kidney is not able to produce secondary urine from the injected primary urine. This can, for example, be the case when the tubules of the kidney are not fully functional any more. However, it can still be advantageous to bring the kidney in contact with the primary urine. The contact of the primary urine with an internal cell surface of the proximal tubules cells, typically enables support of metabolism of middle and high molecular weight uremic toxins. In another realization, the fluid is, additionally or alternatively, extracted from the sealing member as described above. This is advantageous, in the case that fluid leaks from the kidney.

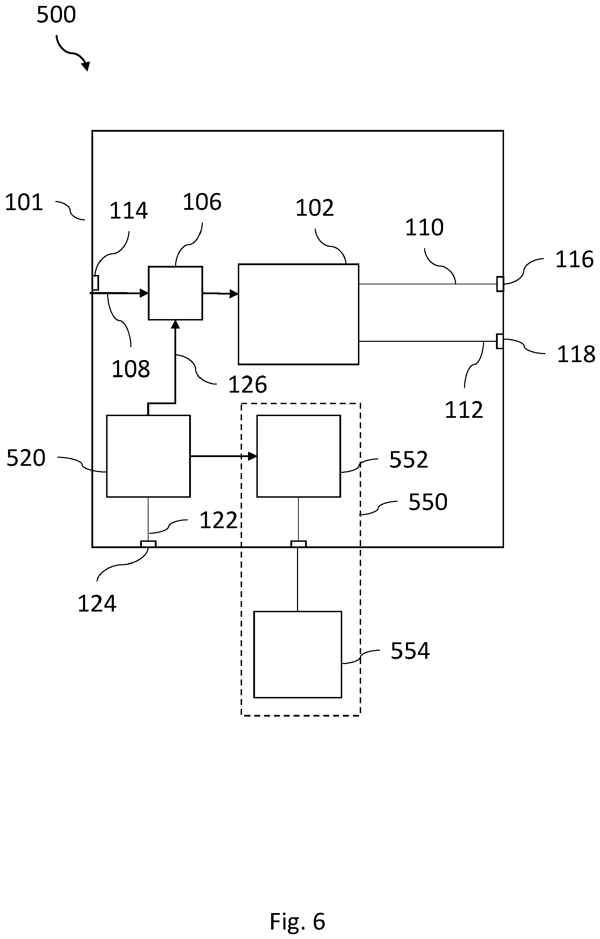

In the following, a medical system will be described with reference to Fig. 6 in which the medical system comprises additional means to further support the patient's kidney in taking up the primary urine.

Fig. 6 shows a medical system 500 that comprises an ultrasound unit.

The medical system 500 comprises an ultrasound unit 550 including an ultrasound control unit 552 and an ultrasound head 554, wherein the ultrasound probe head is configured to be placed at the patient's kidney. Furthermore, the medical system 500 comprises a control unit 520 that is configured to activate the ultrasound unit 550 such that the ultrasound head 554 emits ultrasound waves for widening paracellular pathways by vibration within the patient's kidney.

As has already been mentioned above, besides primary urine it can also be advantageous to inject other fluids into the cortex of the patient's kidney. In the following, other medical devices for injecting a fluid into the cortex will be described with reference to Figs. 7-9.

At first, a medical device will be described with reference to Fig. 7 that allows the injection of a fluid that is sourced from a fluid reservoir.

Fig. 7 shows a medical system 600 that uses a fluid reservoir 602 for sourcing the fluid to be injected into the patient's kidney.

The medical system 600 comprises the fluid reservoir 602 for storing the fluid, which is in the case of the medical system 600 a combination of purified isotonic water and/or a medication. The fluid is pumped through a fluid pump assembly 606 comprising a fluid pump from the fluid reservoir to a fluid outlet 616.

Furthermore, the medical system 600 comprises a control unit 620 that receives physiological condition information 622 indicative of a physiological condition of the patient through a condition-information input interface 624 and is configured to control the flow of the fluid through the fluid outlet 616 using the physiological condition information 622. In particular, the control unit 620 is configured, depending on the physiological condition information 622, to generate and provide fluid control information 626 which are received by the fluid pump assembly 606. The fluid pump assembly in turn is configured to control the fluid pump according to the fluid control information.

In certain situations, the amount of fluid injected into the patient's kidney to quickly remove intoxications needs to be removed from the patient's body to prevent overhydration. A medical system that is configured to also remove fluid from the patient's blood stream will be described in the following with reference to Fig. 8.

Fig. 8 shows a medical system 700 comprising a fluid-removal unit 740.

The fluid-removal unit 740 receives a patient's blood through a blood inlet 746 and is configured to separate a part of the fluid from the blood before the fluid is returned to the patient's body through a blood outlet 742. The removed fluid is provided at a removed-fluid outlet 744. How much fluid is removed is controlled by a control unit 720 that is configured to generate and provide removal control information 726 to control the amount of fluid that is removed from the blood in accordance with the amount of fluid injected into the patient's kidney.

In addition, the medical system 700 may or may not comprise a heating and/or cooling unit 704 that receives the fluid from the fluid reservoir and is configured to adjust temperature of the fluid before the fluid leaves the medical system 700.

It can also be advantageous to use an injection of fluid into the kidney in combination with a peritoneal dialysis. A medical system that is configured for this approach will be described in the following with reference to Fig. 9.

Fig. 9 shows a medical system 800 for supporting peritoneal dialysis.

The medical system 800 comprises as the fluid source a peritoneal extraction unit 802 for extracting peritoneal fluid from a patient's peritoneal space, wherein the peritoneal extraction unit 802 is configured to extract peritoneal fluid from a peritoneal inlet 806 and to provide the extracted peritoneal fluid as the fluid to be injected into the patient's kidney at a peritoneal outlet 804. Furthermore, the medical system 800 comprises a fluid-transfer unit 808 for transferring fluid from the cortex of the patient's kidney to the patient's peritoneal space, wherein the transfer unit 808 is configured to extract fluid from the patient's kidney through a transfer inlet 810 and provide that fluid at a transfer outlet 812.

The amount of fluid transferred from the peritoneal space to the kidney and vice versa is controlled by a control unit 820 which receives physiological condition information 822 through a condition-information input interface 824 and is configured to generate and provide fluid control information 826, 827 using to the physiological condition information 822. Furthermore, the peritoneal extraction unit 802 and the fluid-transfer unit 808 receive the fluid control information and are configured to set a flow rate of fluid through the peritoneal extraction unit 802 and the fluid-transfer unit 808 according to the fluid control information 827. The control unit 820 is configured to receive physiological condition information 822 that comprises physiological parameters indicative of the glucose concentration in the patient's blood as well as a patient's blood volume and is configured to generate the fluid control information 826 using the physiological parameters.

In addition to the described medical systems, in the following, complementing methods will be described with reference to Figs. 10-14

Fig. 10 shows an example for a method 900 for supporting native kidney function of a patient's kidney.

The method 900 comprises two steps. In a first step 902, fluid is received from a fluid source. In a second step 904, the received fluid is injected into a cortex of the patient's kidney.

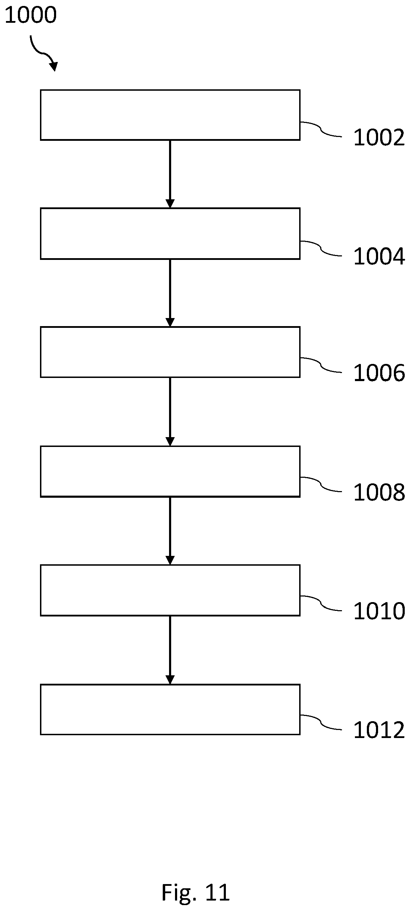

Various fluid sources can be used together with this method. In the following, methods using different fluid sources will be described. At first, methods using a fluid reservoir as the fluid source will be explained in detail. Such a method is shown in Fig. 11.

Fig. 11 shows a method 1000 for supporting native kidney function of a patient's kidney wherein a fluid is received from a fluid reservoir.

The method 1000 comprises in total six steps. In a first step 1002, fluid is received from the fluid reservoir. In a second step 1004, this fluid is pre-heated or pre-cooled. In a third step 1006, the pre-heated fluid is injected into the cortex of the patient's kidney. In a fourth step 1008, blood is drawn from the patient. In a fifth step 1010, fluid is separated from the blood drawn from the patient in accordance with the fluid injected into the cortex. In a sixth step 1012, the blood from which the fluid was separated is returned to the patient's vascular system.

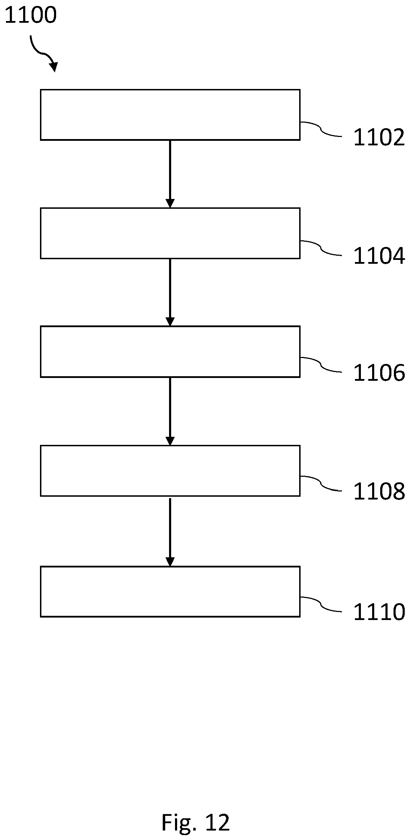

In another embodiment of the method, the method is used in support of a peritoneal dialysis. Such a method is described in the following with reference to Fig. 12.

Fig. 12 shows a method used in support of a peritoneal dialysis.

The method comprises five steps. In a first step 1102, a glucose concentration in the patient's blood is monitored as well as a patient's blood volume. In a second step 1104, an amount of fluid is removed from a peritoneal space. The amount of fluid is determined by the glucose concentration and the patient's blood volume to insure that the glucose concentration in the patient's blood will not reach a level that endangers the patient. One possible choice is to use an amount of peritoneal fluid, which, after injection into the patient's kidney, does not increase the patient's blood glucose level above 10mmol/l. Furthermore, also a blood volume increase of the patient's blood due to the injection of fluid into the cortex is to be prevented. In a third step 1106, the fluid removed from the peritoneal space is injected into the cortex of the patient's kidney. In a fourth step 1108, fluid is removed from the cortex of the kidney in similar quantity as injected. In a fifth step 1110, the fluid removed from the cortex is returned into the peritoneal space.

This method can be used in combination with normal or lower osmolarity peritoneal dialysis, in which dialysate is initially added to the peritoneal space and then periodically exchanged with fresh dialysate. The method will accomplish that the kidney will partially contribute to dialysate clearance of middle and large molecules and it can further be used for fluid excretion if desired. As the trans-peritoneal osmotic gradient - caused by venous return - will be reduced using this method certain osmotic supplement must be added from time to time.

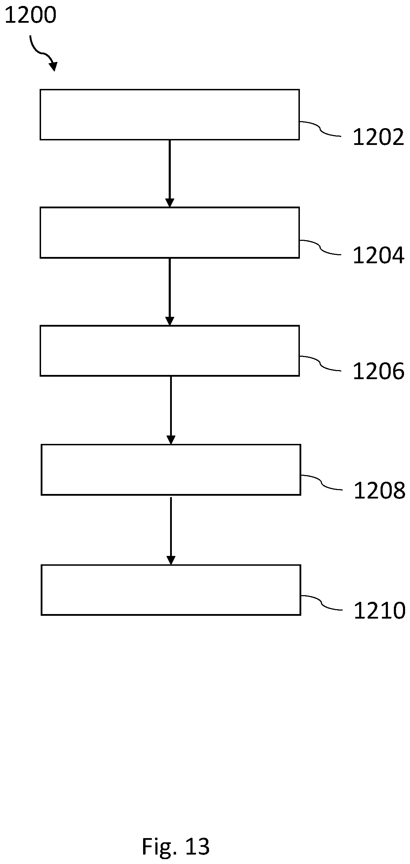

Lastly, in a final embodiment, a source of the fluid may also be primary urine that was separated from the blood of the patient. This method will be explained with reference to Fig. 13.

Fig. 13 shows a method 1200 for supporting native kidney function of a patient's kidney, wherein primary urine is injected into the kidney.

The method comprises in total five steps. In a first step 1202, blood is drawn from the patient. In a second step 1204, primary urine is separated from the blood. In a third step 1206, the primary urine is injected into the cortex of the patient's kidney. The last two steps 1208 and 1210 are optional. In the step 1208, physiological condition information indicative of a physiological condition of the patient's body are received. In the fifth step 1210, a flow of the primary urine injected into the cortex is being controlled using the physiological condition information.

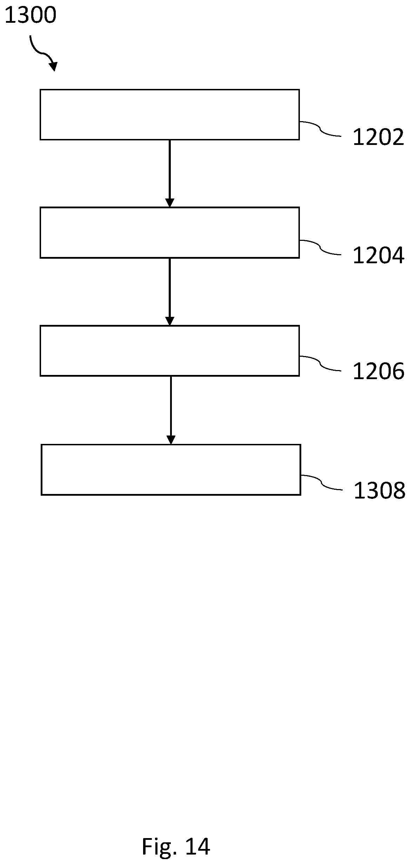

In certain circumstances, it is also advantageous to extract fluid, in particularly fluid partly comprising primary urine, from the patient's body. Such a method is shown in Fig. 14.

Fig. 14 shows a method 1300 that comprises extracting fluid from the patient's body.

The first three steps of the method 1300 are identical to those of method 1200. A third step 1308 of the method 1300 entails extracting fluid from the patient's kidney.

Method 1300 is particularly advantageous in those cases, where a creation of secondary urine from the injected primary urine is not possible for the patient's kidney. This can, for example, be the case when the tubules of the kidney are not fully functional any more. However, it can still be advantageous to bring the kidney in contact with the primary urine. The contact of the primary urine with an internal cell surface of the proximal tubules cells, typically enables support of metabolism of middle and high molecular weight uremic toxins. This can, for example, support regular dialysis. Alternatively, extracting fluid from the patient's kidney can also be helpful, when the tubules are still functioning but reducing secondary urine flow and the native venous outflow is reduced also. In this case, the extraction of fluid can reduce an internal pressure of the kidney while keeping the proximal tubule cells in contact to the primary urine. This can be advantageous, when primary urine is inserted into the kidney at higher rates than the venous system can remove for example, because physiological conditions within the kidney changed. As a result, excess primary urine collects in the kidney. In other methods, the extracted fluid is, additionally or alternatively, injected into the patient's native vascular system.

In summary, the present disclosure describes a medical system (600) for supporting native kidney function of a patient's kidney, which comprises a fluid source (602) for providing fluid to be injected into the patient's kidney, a fluid outlet (616) in fluid connection with the fluid source for transferring the fluid to a renal cortex of the patient's kidney, and a control unit (620) that receives physiological condition information (622) indicative of a physiological condition of the patient and that is configured to control a flow through the fluid outlet (616) using the physiological condition information (622).

## Claims

1. A medical system (600) for supporting native kidney function of a patient's kidney, comprising:
a fluid source (602) for providing fluid to be injected into the patient's kidney,
a fluid outlet (616) in fluid connection with the fluid source for transferring the fluid to a renal cortex of the patient's kidney, and
a control unit (620) that receives physiological condition information (622) indicative of a physiological condition of the patient and that is configured to control a flow through the fluid outlet (616) using the physiological condition information (622).

2. The medical system (600) according to claim 1, wherein the fluid source comprises a fluid reservoir (600), in particular for storing a purified isotonic solution.

3. The medical system (700) according to claim 2, comprising a fluid-removal unit (740) that is configured to remove excess fluid from the patient's blood, wherein the control unit (720) is configured to control an amount of fluid that is removed from the patient's blood by the fluid-removal unit (740) in accordance with the flow of fluid through the fluid outlet (616).

4. The medical system (800) according to claim 1, wherein the fluid source comprises
a peritoneal extraction unit (802) for extracting peritoneal fluid from a patient's peritoneal space, wherein the peritoneal extraction unit (802) is configured to extract peritoneal fluid from a peritoneal inlet (806) and to provide the extracted peritoneal fluid as the fluid to be injected into the patient's kidney, and
a fluid-transfer unit (808) for transferring fluid from the cortex of the patient's kidney to the patient's peritoneal space, wherein the fluid-transfer unit (808) is configured to extract fluid through a renal inlet (810) and provide that fluid at a peritoneal outlet (812).

5. The medical system (100) according to claim 1, wherein the fluid source comprises a separation unit (104) that is configured to separate primary urine (112) from a patient's blood (108) received through a blood inlet (114) and to provide the primary urine (112) at the fluid outlet (118).

6. The medical system (400) according to claim 5, comprising:
a renal extraction unit (442) for extracting renal fluid from a patient's kidney, wherein the renal extraction unit is configured to extract renal fluid from an extraction inlet (446).

7. The medical system (400) of claim 6, wherein the renal extraction unit (442) is configured to provide the extracted renal fluid at an outlet that is connectable to a vessel of a patient's native vascular system for discharging it into the patient's blood stream.

8. The medical system (100) according to any of the claims 5 to 7, wherein the separation unit (104) comprises a filter unit for separating the primary urine (112) from the blood (108) through hemofiltration.

9. The medical system (200) according to any of the preceding claims, comprising a fluid conduit unit (236) with a connection end that is in fluid connection to the fluid outlet (118) and an output end that is insertable into the renal cortex of the patient's kidney for discharging the fluid (108) into the renal cortex of the patient's kidney.

10. The medical system (200) according to claim 9, wherein the output end of the fluid conduit unit (236) comprises a securing member (240A, 240B) that is, when the output end is inserted into the renal cortex, configured to hold the output end in place and/or reduce leakage of fluid from the patient's kidney surrounding the output end.

11. The medical system (200) according to any of the preceding claims comprising a sealing member (300) that is configured to at least partly enclose a renal capsule of the patient's kidney to prevent an escape of fluid from a puncture of the renal capsule.

12. The medical system (200) of any of the preceding claims, comprising a sensing unit (234) that is configured to detect one or more measurement quantities of a patient's body indicative of the physiological condition of the patient and to generate, using the one or more measurement quantities, the physiological condition information and provide the physiological condition information to the control unit.

13. The medical system (200) of any of the preceding claims, comprising:
an additive storage unit (242) for storing one or more additives; wherein
the medical system (200) is configured to add a portion of the one or more additives to the fluid (112) before the fluid is transferred to the renal cortex of the patient's kidney.

14. The medical system (200) of any of the preceding claims, comprising an electrical-energy provision unit (232) that is configured to provide electrical energy to be used for operating the medical system (200).

15. The medical system (500) of any of the preceding claims, comprising an ultrasound unit (550) including an ultrasound probe head, wherein the ultrasound probe head is configured to be placed at the patient's kidney; and wherein
the control unit (520) is configured to activate the ultrasound unit (550) such that the ultrasound unit emits ultrasound waves for widening narrow fluid paths within the kidney by sonic vibration.
